# EUROPEAN PATENT APPLICATION

(11) **EP 3 779 442 A1**
(43) Date of publication of application: **17.02.2021**
(21) Application number: 19776290.9
(22) Date of filing: 08.01.2019
(51) Int. Cl.: G01N 33/542, C12M 1/34, C12Q 1/02, C12Q 1/26, G01N 21/64, C07K 16/00, C12N 9/02, C12N 11/00, C12N 15/115

(54) **TARGET SUBSTANCE DETECTION METHOD, TARGET SUBSTANCE DETECTION KIT, AND TARGET SUBSTANCE DETECTION APPARATUS**

(30) Priority: 28.03.2018 JP 2018061893
(71) Applicant: Sony Corporation, 108-0075 Tokyo (JP)
(72) Inventor: HOSOKAWA, Hiroyuki, Tokyo 108-0075 (JP); ABE, Tomoteru, Tokyo 108-0075 (JP)
(74) Representative: D Young & Co LLP
(86) International application number: PCT/JP2019/000133
(87) International publication number: WO 2019/187467

(57) **Abstract**

To provide a method for detecting a target substance by using an interaction through a molecular species having low reactivity, with two types of microparticles as proximity probes.

A target substance detection method includes the following steps of:
a step (A) of bringing a biological sample into contact with an oxygen sensor bound to a first molecule that specifically binds to a target substance;
a step (B) of bringing the biological sample into contact with a first solid phase support that holds a second molecule and an oxygen-consuming enzyme, the second molecule specifically binding to the target substance; and
a step (C) of acquiring information generated from the oxygen sensor by the steps (A) and (B).

## Description

### TECHNICAL FIELD

The present invention relates to a target substance detection method, a target substance detection kit, and a target substance detection device.

### BACKGROUND ART

A method for utilizing a detection probe has conventionally been used widely to detect a target substance (protein, peptide, nucleic acid, small molecule, complex thereof, or the like). Examples thereof include a method for distinguishing between a detection probe that is bound to a target substance and a detection probe that is not bound to a target substance by utilizing binding to the bottom surface of a microwell plate.

Examples of generally used methods include a sandwich ELISA method. The sandwich ELISA method uses two types of antibodies each of which specifically binds to a target substance. One antibody (capture antibody) is bound to the bottom surface of the microwell plate to capture the target substance. Thereafter, the other antibody (detection antibody) labeled with an enzyme is bound to the target substance bound to the bottom surface of the microwell plate through the one antibody. By performing activity measurement of the amount of the enzyme bound to the bottom surface of the microwell plate, the amount of the target substance contained in the biological sample solution can be measured. Furthermore, detection using two types of antibodies provides a detection method with highly specificity.

In the sandwich ELISA method, it is important to cause binding to the bottom surface of the microwell plate, and to remove unbound substances. This is because, in each experimental step, carrying over of the unbound target substance and enzyme-labeled antibody in the microwell plate to the next step, or the enzyme-labeled antibody non-specifically adsorbed on the bottom surface of the microwell plate becomes a background signal. This is a time-consuming and labor-intensive method because it requires a great deal of cleaning work.

To solve this problem, some detection methods using proximity probes has been developed that do not require a washing step. In the sandwich ELISA method, it can also be said that by measuring the enzyme-labeled antibody bound to the bottom surface, it is detected that two types of antibodies, the antibody bound to the bottom surface and the enzyme-labeled antibody, are in the vicinity.

A method using proximity probes is a method that does not use the bottom surface of the microwell and uses a probe set that is devised so that a labeling signal is generated only when two types of probes are in the vicinity. By being able to distinguish whether or not the two types of probes are in the vicinity, it is possible to allow the presence of unbound probes and reduce the washing step. High specificity can be maintained by using antibodies that are respectively probe-labeled with antibodies corresponding to the complementary antibody and the detection antibody in the sandwich ELISA method.

Examples of the detection method using the proximity probes include Scintillation proximity assay (Mol Immunol 1979), AlphaLISA (Perkin Elmer, Inc.), Proximity Ligation assay (PLA) using nucleic acid, Proximity Extension assay (PEA), and FRET-based assay (Cytometry 1998).

The Scintillation proximity assay uses, as the proximity probes, radioactive atoms and beads containing scintillators that fluoresce due to emitted electrons of radioactive atoms. Since the distance that emitted electrons travel in water is limited, high fluorescence is emitted in a case where the radioactive atoms and the beads containing the scintillator that fluoresce are in the vicinity (Patent Document 1).

In AlphaScreen/AlphaLISA (Perkin Elmer, Inc.), beads that generate singlet oxygen due to a photoreaction and beads that fluoresce due to the singlet oxygen are used as the proximity probes. Only in a case where the singlet oxygen has a half-life of about 4 microseconds, and two types of beads are in the vicinity, energy transfer through the singlet oxygen occurs and fluorescence is emitted (Patent Document 2).

The Proximity Ligation assay (PLA) and the Proximity Extension assay (PEA) use nucleic acids as the proximity probes.

In the Proximity Ligation assay (PLA), single-stranded nucleic acid is used as a probe, and one probe is immobilized on the 5' end side and the other probe is immobilized on the 3' end side. By adding a nucleic acid that hybridizes in such a manner that the 3' end side and the 5' end side of the non-immobilized side are connected together, only the combination of probe nucleic acids existing in the vicinity is bound. After the ligation reaction, the bound nucleic acid can be quantified by using PCR or the like (Patent Document 3).

In the Proximity Extension assay (PEA), a part of the 3' end side and the 5' end side of the non-immobilized side is caused to have a complementary sequence, so that only the combination of probe nucleic acids existing in the vicinity is extended by DNA polymerase (Patent Document 4).

In the FRET-based assay, fluorescent molecules are used as the proximity probes. The fluorescence resonance energy transfer is used that occurs in a case where the fluorescent molecules are in the vicinity (Patent Document 5).

### CITATION LIST

### PATENT DOCUMENT

Patent Document 1: US Pat. No. 4,568,649
Patent Document 2: US Pat. No. 9,393,306
Patent Document 3: US Patent Publication No. 2008/0293051
Patent Document 4: US Pat. No. 7,306,904
Patent Document 5: US Pat. No. 7,615,620

### SUMMARY OF THE INVENTION

### PROBLEMS TO BE SOLVED BY THE INVENTION

By these methods, while maintaining high specificity, the complexity of the washing step can be reduced, but all methods are aimed only at the detection of the target substance contained in the biological sample solution, and do not focus on influence and the like in the case of presence of a biological sample, in particular, living cells.

Since the scintillation proximity assay uses a radioactive isotope, cells and genes may be changed. For a similar reason, the experimental environment is also limited.

The AlphaScreen/AlphaLISA (Perkin Elmer, Inc.) uses energy transfer through the singlet oxygen, but the singlet oxygen has very high reactivity. It is considered that the influence on living cells may occur in the case of use in the presence of the living cells.

In the Proximity Ligation assay (PLA) and Proximity Extension assay (PEA), PCR is used as a detection method, and thus high temperature is used in which the influence on the living cells would occur. In the FRET-based assay, the influence on the living cells is considered to be small, but by the fact that the signal is weak and the distance in which the fluorescent molecules can be used as the proximity probes is less than or equal to 10 nm that is very short, the size of the substance to be detected is limited.

Thus, in the present technology, a method has been developed for detecting a target substance by using an interaction through a molecular species having low reactivity, and using two types of particles as the proximity probes.

### SOLUTIONS TO PROBLEMS

That is, the present technology provides a target substance detection method including the following steps of:
a step (A) of bringing a biological sample into contact with an oxygen sensor bound to a first molecule that specifically binds to a target substance;
a step (B) of bringing the biological sample into contact with a first solid phase support that holds a second molecule and an oxygen-consuming enzyme, the second molecule specifically binding to the target substance; and
a step (C) of acquiring information generated from the oxygen sensor by the steps (A) and (B).
In the step (A), the oxygen sensor may be held on a second solid phase support.
Furthermore, the biological sample preferably contains living cells.
Furthermore, a step (D) of bringing the living cells into contact with a cell stimulating substance can be further included.
The steps (A) to (D) can be performed in a well enabled to capture a single cell.
The target substance can be selected from a group consisting of proteins, peptides, nucleic acids, carbohydrates, lipids, vitamins, and hormones.
The oxygen sensor can be selected from a group consisting of metal porphyrins, metal phthalocyanines, metal chlorins, and ruthenium complexes.
Furthermore, the information generated from the oxygen sensor is optical information, and can be acquired by irradiation with excitation light having a wavelength range corresponding to the oxygen sensor.
The information generated from the oxygen sensor can be phosphorescence intensity or fluorescence intensity. The phosphorescence intensity or fluorescence intensity may be acquired over time.
Furthermore, oxidase can be used as the oxygen-consuming enzyme.
Furthermore, it is preferable that the second solid phase support is a plate or a particle, and the first solid phase support is a particle.
Moreover, the first molecule that specifically binds to the target substance and the second molecule that specifically binds to the target substance can be molecules including antibodies, aptamers, or molecular recognition polymers.

The present technology can also provide a target substance detection kit including:
a first solid phase support that holds a second molecule and an oxygen-consuming enzyme, the second molecule specifically binding to a target substance; and
a second solid phase support that holds a first molecule and an oxygen sensor, the first molecule specifically binding to the target substance.

The present technology can further provide a target substance detection device including:
a microchip mounting unit that mounts a microchip including a well into which a biological sample is put;
a sample flow control unit that controls a flow of the biological sample;
a first contact portion that brings the biological sample into contact with an oxygen sensor bound to a first molecule that specifically binds to a target substance;
a second contact portion that brings the biological sample into contact with a second solid phase support that holds a second molecule and an oxygen-consuming enzyme, the second molecule specifically binding to the target substance;
a light irradiation unit that irradiates the well of the microchip with light; and
an information acquisition unit that acquires information generated from the oxygen sensor.

### EFFECTS OF THE INVENTION

By using an interaction through a molecular species having low reactivity, an assay can be performed in the presence of living cells and the like without limitation of the size of the target substance.

Note that, the effect described here is not necessarily limited, and can be any effect described in the present disclosure.

### BRIEF DESCRIPTION OF DRAWINGS

Fig. 1 is a graph illustrating a relationship between oxygen concentration and phosphorescence intensity of an oxygen sensor.
Fig. 2 is a schematic diagram illustrating a bead coated with an oxygen sensor and having first molecules immobilized thereon.
Fig. 3 is a schematic diagram when phosphorescence is generated by irradiating the bead with excitation light, the bead having the oxygen sensor and the first molecules immobilized thereon.
Fig. 4 is a schematic diagram illustrating a bead having second molecules and oxygen-consuming enzymes that are immobilized thereon.
Fig. 5 is a schematic diagram illustrating action of the bead having second molecules and oxygen-consuming enzymes that are immobilized thereon.
Fig. 6 is a schematic diagram illustrating detection of a target substance of the present technology.
Fig. 7 is a schematic diagram illustrating an outline of a target substance detection device.
Fig. 8 is a schematic diagram of an example in which a protein secreted from a single cell is measured by using a single cell chip with the present technology.
Fig. 9 is a schematic diagram illustrating a detection system of Reference Example 1.
Fig. 10 is a photograph substituting a drawing illustrating a result of Reference Example 1.
Fig. 11 is a schematic diagram illustrating a single cell chip used in Reference Example 2.
Fig. 12 is a diagram illustrating a result of Reference Example 2.

### MODE FOR CARRYING OUT THE INVENTION

The following is a description of preferred embodiments for carrying out the present technology. Note that, the embodiments described below are representative embodiments of the present technology, and the scope of the present technology should not be construed narrowly. Note that, description will be made in the following order.
1. Target substance detection method
   1-1. Biological sample
   1-2. First molecule and second molecule
   1-3. Oxygen sensor
   1-4. Oxygen-consuming enzyme
   1-5. Steps of detection method
2. Target substance detection kit
   2-1. First solid phase support
   2-2. Second solid phase support
3. Target substance detection device
4. Embodiments
   4-1. Embodiment 1
   4-2. Embodiment 2
   4-3. Embodiment 3
   4-4. Embodiment 4
   4-5. Embodiment 5
5. Reference Examples
   5-1. Reference Example 1
   5-2. Reference Example 2

### <1. Target substance detection method>

### 1-1. Biological sample

In the present technology, a biological sample is not particularly limited, and examples thereof includes a single cell-containing sample in which cells are singled and suspended in a buffer or the like, a nucleic acid-containing sample, a cell secretion-containing sample, a cell extract, a cell culture medium, and body fluids such as urine, blood, blood plasma, serum, spinal fluid, and lymph.

### 1-2. First molecule and second molecule

A first molecule and a second molecule are molecules that specifically react with a target substance contained in the biological sample.

Specific examples include antibodies, aptamers, nucleic acids, and molecular recognition polymers.

As the antibodies, it is possible to use those prepared by using mouse, rabbit or the like, with a target substance as an antigen. Examples thereof include antibodies to CD antigens that appear on the cell surface upon differentiation, antibodies to various cancer-specific antigens, antibodies to major histocompatibility antigens, antibodies to sugar chains, antibodies to various cell secretions, antibodies to various proteins and peptides, and the like.

The aptamers are nucleic acid molecules or peptides that specifically bind to a target substance. Examples thereof include DNA aptamers, RNA aptamers, peptide aptamers, modified aptamers in which modification is introduced into a nucleic acid skeleton or a base to improve specificity, and the like.

The molecular recognition polymers capture a target cell surface molecule with high selectivity even in the presence of a compound having physicochemical properties similar to a target substance. The molecular recognition polymers are also called molecular imprinted polymers, and have compound recognition regions that are selectively synthesized.

Note that, the first molecule and the second molecule specifically bind to the same target substance, but a part of the target substance recognized by the first molecule and a part of the target substance recognized by the second molecule are preferably different from each other.

Note that, the target substance for the first molecule and the second molecule is not particularly limited, and examples thereof include biological molecules such as proteins, peptides, nucleic acids, carbohydrates, lipids, vitamins, and hormones.

### 1-3. Oxygen sensor

An oxygen sensor can be selected from a group consisting of metal porphyrins, metal phthalocyanines, metal chlorins, and ruthenium complexes.

Examples of the metal of the metal porphyrin complex include platinum, magnesium, zinc, cadmium, palladium, iron, copper, cobalt, manganese, vanadium, nickel, rhodium, iridium, and the like. In the present technology, the metal introduced into porphyrin is not particularly limited, but platinum can be preferably used, for example.

Examples of the platinum porphyrin complex include platinum octaethylporphyrin (PtOEP), platinum octaethylporphyrin ketone (PtOEPK), platinum tetrakis (pentafluorophenyl) porphyrin (PtTFPP), platinum tetra (N methyl-4-pyridyl) porphyrin (PtTMP), platinum tetraphenyl porphyrin (PtTPP), platinum tertiary butyl phenyl porphyrin (PtTDBPP), platinum tetradichlorophenyl porphyrin (PtTDCPP), platinum tetradibromophenyl porphyrin (PtTBCPP), and the like. In the present technology, the platinum porphyrin complex is not particularly limited, but platinum octaethylporphyrin (PtOEP) can be preferably used, for example.

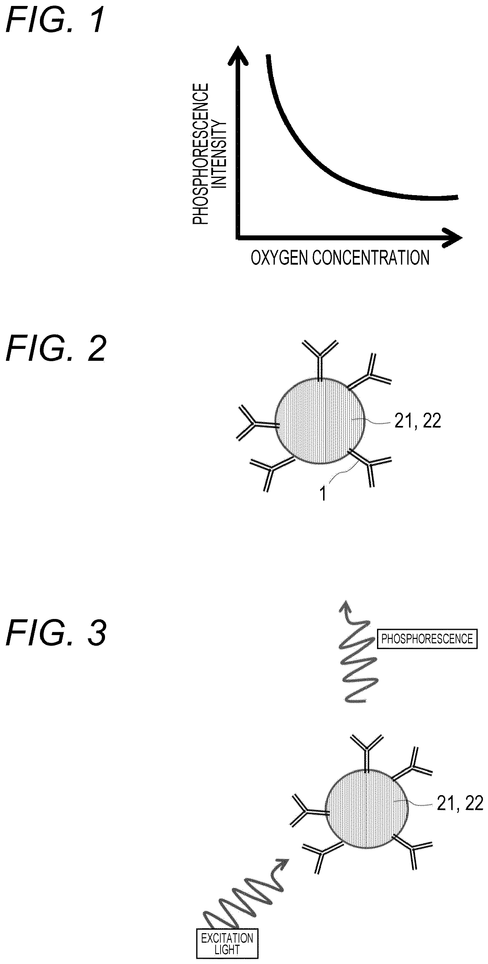

Platinum octaethylporphyrin (PtOEP) emits phosphorescence when excited by UV light or a 405 nm laser. It is known that the phosphorescence lifetime and intensity change depending on the oxygen concentration, and in a case where the oxygen concentration is low, the phosphorescence lifetime extends and the intensity also increases (Fig. 1).

Furthermore, for example, since platinum tetraphenyl porphyrin (PtTPP) and ruthenium complex are also oxygen-sensitive fluorescent substances, for example, when the oxygen-sensitive substance is excited by 480 nm light irradiation, and transitions from the excited state to the ground state, fluorescence with, for example, a wavelength of 610 nm is emitted, but the fluorescence is quenched in the presence of oxygen depending on the concentration of the oxygen.

### 1-4. Oxygen-consuming enzyme

An oxygen-consuming enzyme is specifically an enzyme called oxidase, and examples thereof include polyphenol oxidase, glucose oxidase, lactate oxidase, alcohol oxidase, sulfite oxidase, urease, peroxidase, and the like. In the present technology, the type of oxidase is not particularly limited, but those are preferable that do not give an influence such as denaturation on the target substance or the biological sample.

### 1-5. Steps of detection method

The present technology includes the following steps of:
a step (A) of bringing a biological sample into contact with an oxygen sensor bound to a first molecule that specifically binds to a target substance;
a step (B) of bringing the biological sample into contact with a first solid phase support that holds a second molecule and an oxygen-consuming enzyme, the second molecule specifically binding to the target substance; and
a step (C) of acquiring information generated from the oxygen sensor by the steps (A) and (B), and
can preferably include a step (D) of bringing the living cells into contact with a cell stimulating substance. Note that, the step (A) and the step (B) can be in any order, and the step (B) may be performed first, or the step (A) and the step (B) may be performed at the same time.

In the step (A), an oxygen sensor bound to the first molecule is used. In the present technology, a bead or a plate on which the oxygen sensor bound to the first molecule is immobilized is referred to as "second solid phase support".
Fig. 2 illustrates a bead 22 (second solid phase support) coated with an oxygen sensor 21 and having first molecules 1 immobilized thereon.

By bringing the oxygen sensor bound to the first molecule into contact with the biological sample, the target substance in the biological sample is specifically bound to the first molecule. At this time, incubation may be appropriately performed and stirring may be performed. The incubation temperature, time, and the like can be changed depending on a target cell and the first molecule.

Note that, as illustrated in Fig. 3, when the bead 22 coated with the oxygen sensor 21 and having the first molecules 1 immobilized thereon is irradiated with excitation light, phosphorescence is emitted by the oxygen sensor 21. The oxygen sensor depends on the oxygen concentration, and phosphorescence intensity increases at low concentration (Fig. 1).

In the step (B), the first solid phase support is used that holds the second molecule and the oxygen-consuming enzyme. In the present technology, a bead or a plate on which the second molecule and the oxygen-consuming enzyme are immobilized is referred to as "first solid phase support".

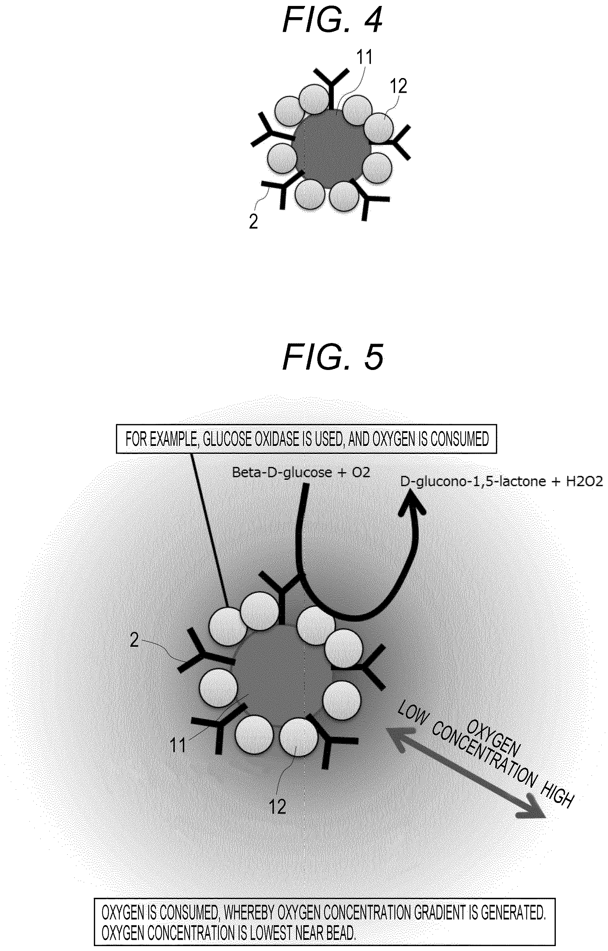

Fig. 4 illustrates a bead 11 (first solid phase support) that holds second molecules 2 and oxygen-consuming enzymes 12.

The target substance specifically bound to the oxygen sensor bound to the first molecule in step (A) specifically binds to the second solid phase support on which the second molecule and the oxygen-consuming enzyme are immobilized. That is, the first molecule and the second molecule forms a sandwich of the target substance. At this time, the incubation may be performed with stirring. The incubation temperature, time, and the like can be changed depending on a target cell and the first molecule.

Furthermore, Fig. 5 illustrates action of the bead 11 having the second molecules 2 and the oxygen-consuming enzymes 12 that are immobilized thereon. Since a large amount of glucose oxidase that is an example of the oxygen-consuming enzyme 12 is immobilized on the bead 11, a low oxygen state is created around the bead 11.

In the step (C), a step is performed of acquiring the information generated from the oxygen sensor by the steps (A) and (B). The information generated from the oxygen sensor is optical information, and is acquired by irradiation with light having a wavelength corresponding to the oxygen sensor. Specifically, the information is, for example, phosphorescence and fluorescence, and their intensities. The information may be acquired by a phosphorescence/fluorescence measuring device or an image, and is not particularly limited.

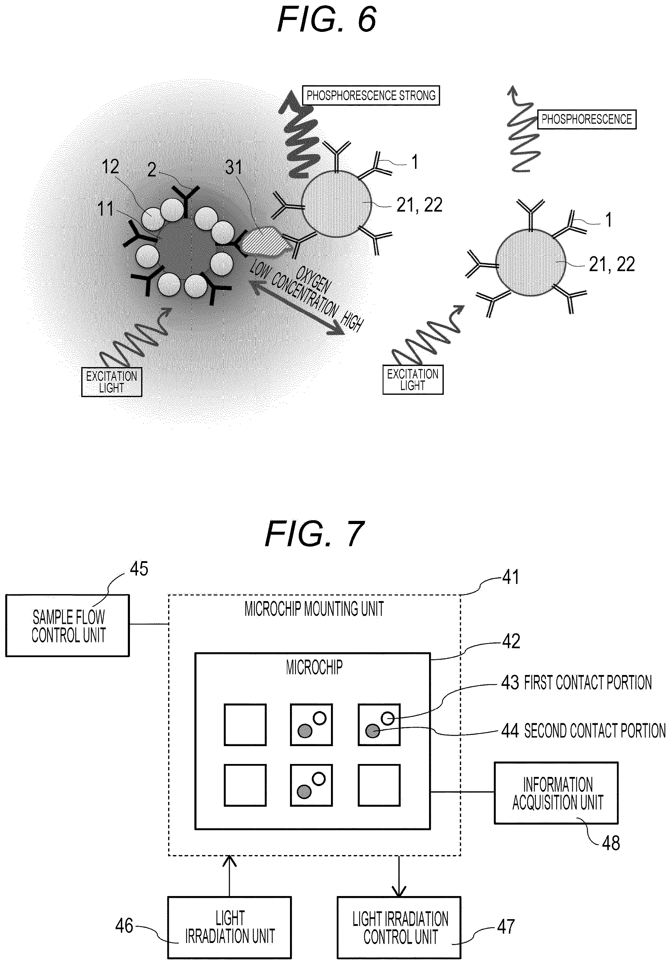

As illustrated on the right side of Fig. 6, when the bead 22 coated with the oxygen sensor 21 and having the first molecules 1 immobilized thereon does not react with a target substance 31, the phosphorescence intensity is weak even when the bead 22 is irradiated with excitation light.

However, as illustrated on the left side of Fig. 6, when the bead 22 coated with the oxygen sensor 21 and having the first molecules 1 immobilized thereon reacts with the target substance 31, and the bead 11 that holds the second molecules 2 and the oxygen-consuming enzymes 12 reacts with the target substance 31, and a sandwich of the target substance 31 is formed, the bead 22 coated with the oxygen sensor 21 and having the first molecules 1 immobilized thereon exists near the bead 11 that holds the second molecules 2 and the oxygen-consuming enzymes 12. Since the oxygen concentration around the bead 11 is lowered due to the oxygen-consuming enzymes 12, phosphorescence is emitted stronger than that from the bead on the right side of Fig. 6 when the bead is irradiated with excitation light.

Furthermore, when the biological sample is living cells, the present technology may further include the step (D) of bringing the living cells into contact with a cell stimulating substance.

Examples of the cell stimulating substance include sugars, amino acids, fats, acids/alkalis (pH adjusters), and vary depending on the type of cells, the type of secretions, and the like.

The contact with the cell stimulating substance may be brought in any of the steps (A) and (B), or may be brought between the steps.

The steps (A) to (D) can be performed in the same container, and the assay can be performed without performing a washing operation. Furthermore, when the biological sample is a living cell and a single cell, the amount of the secretion from the single cell and the like can be measured by performing the steps in a well enabled to capture a single cell. Furthermore, by performing measurement over time, the secretion producing ability of the single cell and the like can be measured.

Note that, when the container is in an open state, the oxygen concentration of the entire solution is in equilibrium with the oxygen concentration in the atmosphere, and the low oxygen state exists only in a very limited region around a microparticle. Since oxygen necessary for cell growth and the like is always present, it is considered that the influence on cells is small.

### <2. Target substance detection kit>

A target substance detection kit includes: a first solid phase support that holds a second molecule and an oxygen-consuming enzyme, the second molecule specifically binding to a target substance; and a second solid phase support that holds a first molecule and an oxygen sensor, the first molecule specifically binding to the target substance. In addition to this, a cell stimulating substance-containing reagent, a biological sample preparation buffer, a disposable container into which the biological sample is put, and the like may be included.

### 2-1. First solid phase support

The first solid phase support holds the second molecule that specifically binds to the target substance, and the oxygen-consuming enzyme. The first solid phase support is not particularly limited, but is preferably a bead, or a microbead.

For example, when the second molecule is an antibody and the oxygen-consuming enzyme is glucose oxidase, the first solid phase support (a bead, a plate, or the like) on which the antibody and the glucose oxidase are immobilized is prepared in advance. The antibody and the glucose oxidase may be directly immobilized on the bead or the plate, or the bead or the plate may be coated with the glucose oxidase in advance and the antibody may be immobilized thereon, or vice versa.

A bead portion where the antibody and the glucose oxidase are not immobilized is coated with a protein, peptide, or the like by using a conventional technology so that a non-specific reaction with the biological sample, the target substance, or the like does not occur.

### 2-2. Second solid phase support

The second solid phase support holds the oxygen sensor bound to the first molecule that specifically binds to the target substance. The second solid phase support is not particularly limited, but is preferably a plate, a bead, or a microbead. The second solid phase support of the bead or the plate may be first coated with the oxygen sensor, and then the first molecule may be immobilized thereon.

A plate portion or a bead portion where the antibody and PtOEP are not immobilized is coated with a protein, a peptide, or the like by using a conventional technology so that a non-specific reaction with the biological sample, the target substance, or the like does not occur.

### <3. Target substance detection device>

As illustrated in Fig. 7,
a target substance detection device of the present technology includes:
a microchip mounting unit 41 that mounts a microchip including a well into which a biological sample is put;
a sample flow control unit 45 that controls a flow of the biological sample;
a first contact portion 43 that brings the biological sample into contact with an oxygen sensor bound to a first molecule that specifically binds to a target substance;
a second contact portion 44 that brings the biological sample into contact with a second solid phase support that holds a second molecule and an oxygen-consuming enzyme, the second molecule specifically binding to the target substance;
a light irradiation unit 46 that irradiates the well of the microchip with light; and
an information acquisition unit 48 that acquires information generated from the oxygen sensor.

A microchip 42 including the well into which the biological sample is put may be disposable, or may be one repeatedly used for measurement. By measuring a single cell over time with a single device, changes in the amount of secretion and the like of a cell product can be tracked. Thus, it is possible to analyze the production capacity and the like of the cells on the basis of the data of the change over time in the amount of secretion from the cells. The cell may be a single cell or a cell mass in which a plurality of cells gathers. In the case of a single cell, the production capacity can be analyzed for an individual cell, and as a result of the analysis, it is possible to select, for example, a cell having high production capacity, and perform further identification, analysis, proliferation, efficient production of the cell product and extraction thereof, and the like.

The sample flow control unit 41 that controls the flow of the biological sample can cause a buffer, a biological sample, or the like to flow from, for example, an external flow channel, and generate force for drawing or force for putting in by a valve, a liquid feeding device, or the like. As a result, a single cell that is a biological sample may be put in a well of a single cell chip, or the sample flow control unit 41 can be made common and the cell stimulating substance reagent or the like can be put in the well. The sample flow control unit 41 may be controlled by a program installed in a computer.

The first contact portion 43 brings the biological sample into contact with the oxygen sensor bound to the first molecule that specifically binds to the target substance, and the first contact portion 43 is, for example, a bead that holds the first molecule and the oxygen sensor, and the contact itself is performed in the well of the microchip. Alternatively, the first molecule and the oxygen sensor may be solid-phased on the well of the microchip.

Furthermore, the second contact portion 44 brings the biological sample into contact with the second solid phase support that holds the second molecule and the oxygen-consuming enzyme, the second molecule specifically binding to the target substance, and the second contact portion 44 is, for example, a bead that holds the second molecule and the oxygen-consuming enzyme, and the contact itself is performed in the well of the microchip 42.

The light irradiation unit 47 that irradiates the well of the microchip 42 with light can change the wavelength of excitation light for irradiation depending on the oxygen sensor used. The light irradiation unit 47 may irradiate the entire microchip, or may irradiate only the well into which the cell is input, for example. Furthermore, it is possible not only to perform irradiation once but also to perform irradiation continuously over time. The irradiation pattern may be controlled by providing a light irradiation control unit 47.

As the information acquisition unit that acquires information generated from the oxygen sensor, it is possible to use a commercially available plate reader, a phosphorescence/fluorescence measuring device, an image acquisition device, and the like. The information acquisition unit can also analyze the information obtained by the information acquisition unit by communicating with an analysis unit. The analysis unit can calculate the secretion producing ability of a single cell on the basis of the obtained data, for example. The data and analysis results obtained here may be stored in a storage unit further installed.

### 4. Embodiments

Hereinafter, embodiments will be described that use a single cell as a biological sample.

### 4-1. Embodiment 1

### [Amount of interleukin-2 secreted by Jurkat cells using SBS standard microplate]

(1) Jurkat cells are seeded in a volume of 4 × 10⁵ cells/mL × 50 µL/1 well on a 96-well microplate.
(2) 50 µL/well of 8 µg/mL Phytohemagglutinin (PHA) or a medium (negative control) is added.
(3) Cultivation is performed under conditions of 37°C and 5% CO₂ for 1 day.
   Culture solutions in which the concentration of purified interleukin-2 (IL-2) are changed and dissolved are prepared as standard samples, and a calibration curve is prepared.
(4) Microparticles containing an oxygen sensor holding anti-IL-2 antibody is added to a cell culture medium.
(5) Incubation is performed for 30 minutes with thorough mixing.
(6) Microparticles containing an oxygen-consuming enzyme holding an anti-IL-2 antibody that recognizes a part different from the above (4) is added to a cell culture medium.
(7) Incubation is performed for 30 minutes with thorough mixing.
(8) Phosphorescence intensity is measured with a plate reader.

### 4-2. Embodiment 2

### [Quantification of amount of IL-2 secreted by Jurkat cells using SBS standard microplate (short version)]

(1) Jurkat cells are seeded in a volume of 4 × 10⁵ cells/mL × 50 µL/1 well on a 96-well microplate.
(2) 50 µL/well of 8 µg/mL Phytohemagglutinin (PHA) or a medium (negative control) is added.
(3) Cultivation is performed under conditions of 37°C and 5% CO₂ for 1 day.
   Culture solutions in which the concentration of purified IL-2 are changed and dissolved are prepared as standard samples, and a calibration curve is prepared.
(4) Microparticles containing an oxygen sensor holding an anti-IL-2 antibody, and microparticles containing an oxygen-consuming enzyme holding an anti-IL-2 antibody that recognizes another part are added to a cell culture medium.
(5) Incubation is performed for 30 minutes with thorough mixing.
(6) Phosphorescence intensity is measured with a plate reader.

### 4-3. Embodiment 3

### [Quantification of amount of IL-2 secreted by Jurkat cells using PDMS chip having single cell wells]

(1) Jurkat cells are seeded on a PDMS chip having single cell wells.
(2) Incubation is performed for 10 minutes.
(3) Microparticles containing an oxygen sensor holding an anti-IL-2 antibody, and microparticles containing an oxygen-consuming enzyme holding an anti-IL-2 antibody that recognizes another part are simultaneously added to 8 µg/mL Phytohemagglutinin (PHA) or a medium (negative control). With this solution, excess cells are washed away, and Jurkat cells are stimulated.
(4) Cultivation is performed under conditions of 37°C and 5% CO₂.
(5) Phosphorescence intensity of each well is measured every hour.

Culture solutions in which the concentration of purified IL-2 are changed and dissolved are prepared as standard samples, and a calibration curve is prepared.

### 4-4. Embodiment 4

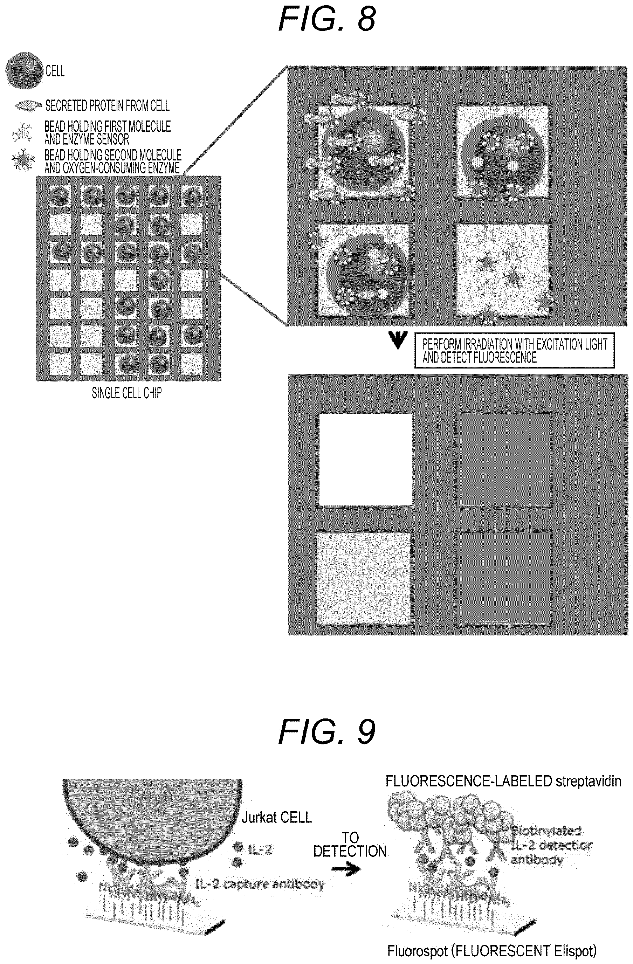

Fig. 8 illustrates a schematic diagram of an example in which a protein secreted from a single cell is measured by using a single cell chip with the present technology.

A single cell is put in each well of the single cell chip, and a bead that holds the oxygen sensor and the first molecule that specifically binds to a specific portion of the secreted protein of the target substance, and a bead that holds the oxygen-consuming enzyme and the second molecule that specifically binds to another specific portion of the secreted protein of the target molecule are introduced into the well. When there is no single cell in the well, there is no secreted protein, and the beads exist separately. Furthermore, the beads exist separately when the secreted protein is not secreted even if there is a single cell in the well.

When a small amount of the secreted protein exists, the beads and the secreted protein forms a sandwich, and the vicinity of the bead holding the oxygen-consuming enzyme is in a low oxygen state, so that weak phosphorescence is generated corresponding to the small amount of the secreted protein when the oxygen sensor is irradiated with excitation light.

When a large amount of the secreted protein exists, the sandwich is formed, and when the oxygen sensor is irradiated with excitation light, strong phosphorescence is generated corresponding to the large amount of protein.

This can be acquired as image data.

### 5. Reference Examples

In reference examples, methods have been performed in which a biotin-streptavidin detection system is used that is a conventional technology, and the secretion from cells is captured and detected with an antibody immobilized on the bottom surface of a culture plate.

### 5-1. Reference Example 1

In Reference Example 1, a system has been performed in which an anti-IL-2 antibody is solid-phased on the bottom surface of a well, a single cell is introduced into the well, and IL-2 secreted from the single cell is detected by sandwiching it between streptavidin-labeled anti-IL-2 antibodies (Fig. 9).

(1) A Jurkat cell suspension is placed on a chip that has a well of a size for putting in a single cell and on which an anti-IL-2 antibody has been solid-phased.
(2) Incubation is performed for about 10 minutes.
(3) PHA/PMA-containing medium is poured in, excess cells are washed away, and Jurkat cells are activated (IL-2 secretion is induced) for 4 hours.
(4) Incubation is performed for about 3 hours.
(5) Biotinylated anti-IL-2 antibodies for detection are poured in.
(6) Excess antibodies are washed away.
(7) Fluorescence-labeled streptavidin is poured in.
(8) Excess streptavidin is washed away.
(9) Observation is performed with a fluorescence microscope.

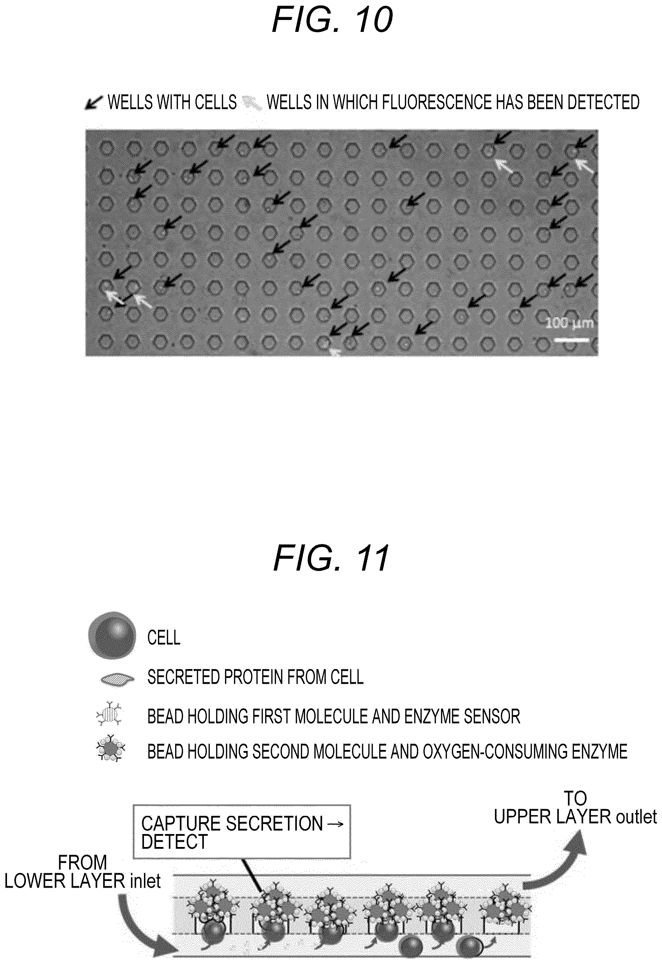

The result is illustrated in Fig. 10.

Black arrows indicate wells with cells, white arrows indicate wells in which fluorescent spots have been detected, and IL-2 secretion from Jurkat cells has been detected. However, there have been cases where IL-2 can be detected even in wells in which no cells exists. The reason is considered that it is necessary to wash the antibody or the like not bound to the bottom surface for detection, and even the cells are washed away in the washing step. It has been suggested that the detection method using the proximity probes that does not require the washing step has an advantage that there is no possibility that cells are washed away.

### 5-2. Reference Example 2

Fig. 11 illustrates a reference example using a chip that is a single cell chip and includes a well that is opened downward largely and sandwiched between a lower layer inlet and an upper layer outlet, and in which a single cell is captured from a flow of the lower layer inlet due to suction force through a through hole from the upper layer outlet. See Japanese Patent Application No. 2017-171921 for details of the single cell chip.

(1) Mesoporous beads having a diameter of 3 µm and bound to anti-IL-2 antibody at the through hole and well portions are captured.
(2) Excess beads are washed away.
(3) Jurkat cells are captured.
(4) Excess cells are washed away.
(5) PHA/PMA-containing medium is poured in, and Jurkat cells are activated to induce IL-2 secretion.
(6) Incubation is performed for about 3 hours.
(7) Biotinylated anti-IL-2 antibodies for detection are poured in.
(8) Excess antibodies are washed away.
(9) Fluorescence-labeled streptavidin is poured in.
(10) Excess streptavidin is washed away.
(11) Observation is performed with a fluorescence microscope.

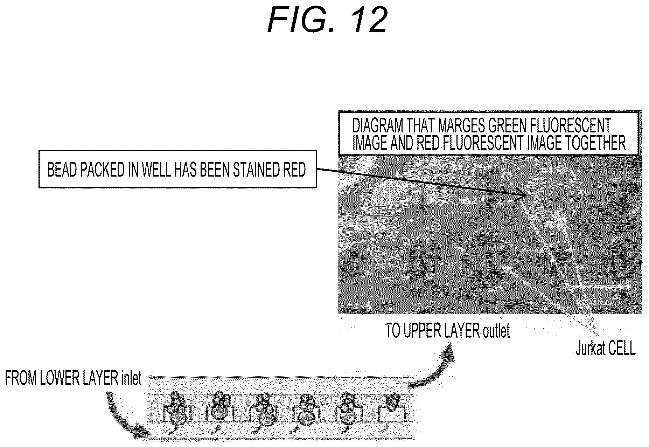

The result is illustrated in Fig. 12.

As illustrated in the upper right of Fig. 12, it has been suggested that there is a possibility that IL-2 secreted by Jurkat cells can be detected in a biotin-streptavidin sandwich detection system that is a conventional detection method, by using the single cell chip of this reference example.

Thus, a possibility has been shown of a detection system using the oxygen sensor and the oxygen-consuming enzyme of the present technology even with the single cell chip of this reference example.

Note that, the present technology can also have the following configurations;
[1] A target substance detection method, including
   the following steps of:
   a step (A) of bringing a biological sample into contact with an oxygen sensor bound to a first molecule that specifically binds to a target substance;
   a step (B) of bringing the biological sample into contact with a first solid phase support that holds a second molecule and an oxygen-consuming enzyme, the second molecule specifically binding to the target substance; and
   a step (C) of acquiring information generated from the oxygen sensor by the steps (A) and (B).
[2] The target substance detection method according to [1], in which in the step (A), the oxygen sensor is held on a second solid phase support.
[3] The target substance detection method according to [1] or [2], in which the biological sample contains living cells.
[4] The target substance detection method according to any of [1] to [3], further including a step (D) of bringing the living cells into contact with a cell stimulating substance.
[5] The target substance detection method according to [4], in which the steps (A) to (D) are performed in a well enabled to capture a single cell.
[6] The target substance detection method according to any of [1] to [5], in which the target substance is selected from a group consisting of proteins, peptides, nucleic acids, carbohydrates, lipids, vitamins, and hormones.
[7] The target substance detection method according to any of [1] to [6], in which the oxygen sensor is selected from a group consisting of metal porphyrins, metal phthalocyanines, metal chlorins, and ruthenium complexes.
[8] The target substance detection method according to any of [1] to [7], in which the information generated from the oxygen sensor is optical information, and is acquired by irradiation with excitation light having a wavelength range corresponding to the oxygen sensor.
[9] The target substance detection method according to any of [1] to [8], in which the information generated from the oxygen sensor is phosphorescence intensity or fluorescence intensity.
[10] The target substance detection method according to [9], in which the phosphorescence intensity or fluorescence intensity is acquired over time.
[11] The target substance detection method according to any of [1] to [10], in which the oxygen-consuming enzyme is oxidase.
[12] The target substance detection method according to any of [2] to [11], in which the second solid phase support is a plate or a particle.
[13] The target substance detection method according to any of [1] to [12], in which the first solid phase support is a particle.
[14] The target substance detection method according to any of [1] to [13], in which the first molecule that specifically binds to the target substance and the second molecule that specifically binds to the target substance are molecules including antibodies, aptamers, or molecular recognition polymers.
[15] A target substance detection kit including:
   a first solid phase support that holds a second molecule and an oxygen-consuming enzyme, the second molecule specifically binding to a target substance; and
   a second solid phase support that holds a first molecule and an oxygen sensor, the first molecule specifically binding to the target substance.
[16] A target substance detection device including:
   a microchip mounting unit that mounts a microchip including a well into which a biological sample is put;
   a sample flow control unit that controls a flow of the biological sample;
   a first contact portion that brings the biological sample into contact with an oxygen sensor bound to a first molecule that specifically binds to a target substance;
   a second contact portion that brings the biological sample into contact with a second solid phase support that holds a second molecule and an oxygen-consuming enzyme, the second molecule specifically binding to the target substance;
   a light irradiation unit that irradiates the well of the microchip with light; and
   an information acquisition unit that acquires information generated from the oxygen sensor.

### REFERENCE SIGNS LIST

- 1: First molecule
- 2: Second molecule
- 11: Bead
- 12: Oxygen-consuming enzyme
- 21: Oxygen sensor
- 22: Bead
- 31: Target substance
- 41: Microchip mounting unit
- 42: Microchip
- 43: First contact portion
- 44: Second contact portion
- 45: Sample flow control unit
- 46: Light irradiation unit
- 47: Light irradiation control unit
- 48: Information acquisition unit

## Claims

1. A target substance detection method, comprising following steps of:
a step (A) of bringing a biological sample into contact with an oxygen sensor bound to a first molecule that specifically binds to a target substance;
a step (B) of bringing the biological sample into contact with a first solid phase support that holds a second molecule and an oxygen-consuming enzyme, the second molecule specifically binding to the target substance; and
a step (C) of acquiring information generated from the oxygen sensor by the steps (A) and (B).

2. The target substance detection method according to claim 1, wherein in the step (A), the oxygen sensor is held on a second solid phase support.

3. The target substance detection method according to claim 1, wherein the biological sample contains living cells.

4. The target substance detection method according to claim 3, further comprising a step (D) of bringing the living cells into contact with a cell stimulating substance.

5. The target substance detection method according to claim 4, wherein the steps (A) to (D) are performed in a well enabled to capture a single cell.

6. The target substance detection method according to claim 1, wherein the target substance is selected from a group consisting of proteins, peptides, nucleic acids, carbohydrates, lipids, vitamins, and hormones.

7. The target substance detection method according to claim 1, wherein the oxygen sensor is selected from a group consisting of metal porphyrins, metal phthalocyanines, metal chlorins, and ruthenium complexes.

8. The target substance detection method according to claim 1, wherein the information generated from the oxygen sensor is optical information, and is acquired by irradiation with excitation light having a wavelength range corresponding to the oxygen sensor.

9. The target substance detection method according to claim 8, wherein the information generated from the oxygen sensor is phosphorescence intensity or fluorescence intensity.

10. The target substance detection method according to claim 9, wherein the phosphorescence intensity or fluorescence intensity is acquired over time.

11. The target substance detection method according to claim 1, wherein the oxygen-consuming enzyme is oxidase.

12. The target substance detection method according to claim 2, wherein the second solid phase support is a plate or a particle.

13. The target substance detection method according to claim 1, wherein the first solid phase support is a particle.

14. The target substance detection method according to claim 1, wherein the first molecule that specifically binds to the target substance and the second molecule that specifically binds to the target substance are molecules including antibodies, aptamers, or molecular recognition polymers.

15. A target substance detection kit comprising:
a first solid phase support that holds a second molecule and an oxygen-consuming enzyme, the second molecule specifically binding to a target substance; and
a second solid phase support that holds a first molecule and an oxygen sensor, the first molecule specifically binding to the target substance.

16. A target substance detection device comprising:
a microchip mounting unit that mounts a microchip including a well into which a biological sample is put;
a sample flow control unit that controls a flow of the biological sample;
a first contact portion that brings the biological sample into contact with an oxygen sensor bound to a first molecule that specifically binds to a target substance;
a second contact portion that brings the biological sample into contact with a second solid phase support that holds a second molecule and an oxygen-consuming enzyme, the second molecule specifically binding to the target substance;
a light irradiation unit that irradiates the well of the microchip with light; and
an information acquisition unit that acquires information generated from the oxygen sensor.
